# EUROPEAN PATENT APPLICATION

(11) **EP 1 921 068 A1**
(43) Date of publication of application: **14.05.2008**
(21) Application number: 06090204.6
(22) Date of filing: 08.11.2006
(51) Int. Cl.: C07D 231/56, C07D 401/04, C07D 405/12, C07D 405/14, A61K 31/41, A61K 31/435, A61P 29/00

(54) **Indazole and indole derivatives as anti-inflammatory agents**

(71) Applicant: Bayer Schering Pharma Aktiengesellschaft, 13353 Berlin (DE); AstraZeneca AB, 151 85 Södertälje (SE)
(72) Inventor: Berger, Markus, 13347 Berlin (DE); Rehwinkel, Hartmut, 10961 Berlin (DE); Schäcke, Heike, 10115 Berlin (DE); Lepistö, Matti, 22742 Lund (SE); Edmann, Karl, 41137 Göteborg (SE)
(74) Representative: Seuss, Thomas

(57) **Abstract**

The present invention relates to the compounds of formula I, 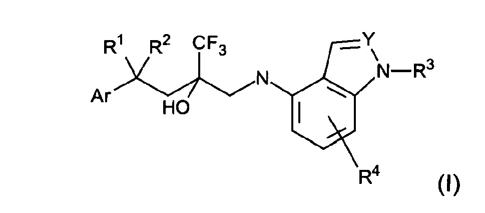 processes for their production and their use as anti-inflammatory agents.

## Description

The present invention relates to compounds of formula I, a process for their production and their use as anti-inflammatory agents.

From the prior art of DE 100 38 639 and WO 02/10143, anti-inflammatory agents of the following general formula 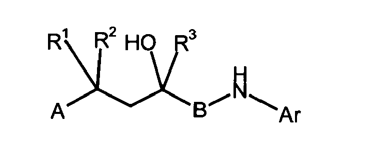 are known, in which the Ar radical comprises phthalides, thiophthalides, benzoxazinones or phthalazinones. In the experiment, these compounds show dissociations of action between anti-inflammatory and undesirable metabolic actions and are superior to the previously described nonsteroidal glucocorticoids or exhibit at least just as good an action.

Further structurally related compounds are known from WO 2006/108699 (Glaxo Group Ltd.). Other prior art compounds are disclosed in WO 2005/003098, WO 2005/030213, WO 03/082280, WO 2004/063163, WO 00/32584, WO 02/10143, WO 03/82827 and DE 10261874.

The selectivity of the compounds of the prior art towards the glucocorticoid receptor (GR) compared to the other steroid receptors still requires improvement, however.

It was therefore the object of this invention to make compounds available whose selectivity towards the glucocorticoid receptor (GR) is improved compared to the other steroid receptors. This object has been achieved by the compounds according to the claims of this application.

This invention therefore relates to stereoisomers of general formula I 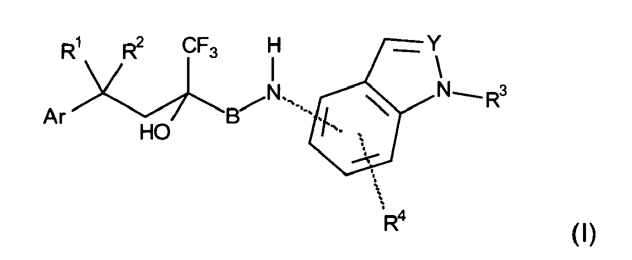 in which
- B: is selected from the group consisting of CH₂ and CO
- Y: is selected from the group consisting of CH and N
- Ar: is selected from the group consisting of a phenyl, a pyridinyl or a pyrimidinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁵, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁵ means hydrogen or C₁-C₄-alkyl
provided that if B is CH₂ and Y is N and R¹, R² are both methyl then Ar is not 2,3-dihydro-1-benzofuran-7-yl, 5-fluoro-2-methoxy-phenyl or 5-fluoro-2-hydroxy-phenyl,
- R¹, R²: are independently selected from the group consisting of a hydrogen atom or a (C₁-C₄)-alkyl group,
or
- R¹, R²: together form a C₃-C₆ cycloalkyl-ring
- R³: is selected from the group consisting of a phenyl, a pyrimidinyl and a pyridinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁶ means hydrogen or C₁-C₄-alkyl
- R⁴: is selected from the group consisting of hydrogen, halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶,
in which R⁶ has the above identified meaning.

Unless otherwise notified the term "alkyl" refers to straight or branched derivatives. For example, the term propyl comprises *ⁿ*-propyl and *^{iso}*-propyl, the term butyl comprises *ⁿ*-butyl, *^{iso}*-butyl and *^{tert}*-butyl.

Unless otherwise notified the term "haloalkyl" refers to straight or branched alkyl derivatives in which at least one hydrogen is substituted by a halogen atom, preferably by a fluoro atom. For example, the term halopropyl comprises perfluoro-*ⁿ*-propyl and perfluoro*^{iso}*-propyl, as well as 1-chloro propyl, the term haloethyl comprises 1-fluoroethyl, 2,2,2- trifluoroethyl, 1-chloroethyl and 2-chloroethyl.

Unless otherwise notifed the term "alkoxy' refers to straight or branched derivatives. For example, the term propoxy comprises *ⁿ*- propoxy and *^{iso}-* propoxy, the term butoxy comrises "-butoxy, *^{iso}*-butoxy and *^{tert}*-butoxy*.*

Unless otherwise notifed the term "halo alkoxy "refers to straight or branched alkoxy derivatives in which at least one hydrogen is substituted by a halogen atom, preferably by a fluoro atom. For example, the term halopropoxy comprises perfluoro-*ⁿ*-propoxy and perfluoro*^{iso}*-propoxy, as well as 1-chloropropoxy, the term haloethoxy comprises 1-fluoroethoxy, 2,2,2- trifluoroethoxy, 1-chloroethoxy and 2-chloroethoxy.

The term halogen atom or halogen means a fluorine, chlorine, bromine or iodine atom. Preferred is a fluorine, chlorine or bromine atom. More preferred is a fluorine or chlorine atom. Most preferred is a fluorine atom.

In one embodiment the invention provides compounds of general formula I, in which Ar is selected from the group comprising:
4-Chloro-2-methoxyphenyl
4-Fluoro-2-methoxyphenyl
5-Chloro-2-methoxyphenyl
4-Chloro-2-hydroxyphenyl
4-Fluoro-2-hydroxyphenyl
5-Chloro-2-hydroxyphenyl
2,3-Dihydrobenzofuran-5-yl
2,3-Dihydrobenzofuran-4-yl
2,3-Dihydro-5-fluorobenzofuran-7-yl
2,3-Dihydro-5-chlorobenzofuran-7-yl
Benzo[1,3]dioxol-4-yl
4-Fluoro-Benzo[1,3]dioxol-4-yl
5-Fluoro-Benzo[1,3]dioxol-4-yl
4-Chloro-Benzo[1,3]dioxol-4-yl
5-Chloro-Benzo[1,3]dioxol-4-yl
2,4-Difluorophenyl
2,5-Difluorophenyl
2-Chloro-5-fluorophenyl
5-Chloro-2-fluorophenyl.

In one embodiment the invention provides compounds of general formula I, in which one of R¹ and R² is methyl and the other is hydrogen. In another embodiment the invention provides compounds of general formula I, in which R¹ and R² together form a cyclopropyl, a cyclobutyl, a cyclopentyl or a cyclohexyl ring. Compounds of general formula I, in which R¹ and R² are both methyl are, however, a preferred subject of the invention.

In one embodiment the invention provides compounds of general formula I, in which B is a -CH₂- group (amines) or
in which B is

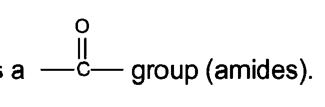

In another embodiment the invention provides compounds of general formula I, in which Y is a nitrogen atom (indazoles) or
in which Y is a CH group (indoles). Preferably Y is a N atom.

The link between the amine or amide and the indazole or indole can be formed via the 4-, 5- or 6-position of the indazole or indole. Preferred are the 4- and the 5-position. Most preferred is the 4-position.

In one embodiment the invention provides compounds of general formula I, in which R³ is selected from the group comprising:
4-fluorophenyl, 3-fluorophenyl, 2-fluorophenyl,
4-chlorophenyl, 3-chlorophenyl, 2-chlorophenyl, 6-fluoropyridin-3-yl,
5-fluoropyridin-3-yl, 4-fluoropyridin-3-yl, 6-chloropyridin-3-yl,
5-chloropyridin-3-yl, 4-chloropyridin-3-yl, 2-fluoropyridin-4-yl,
3-fluoropyridin-4-yl, 2-chloropyridin-4-yl, 3-chloropyridin-4-yl,
4-methylphenyl, 3,5-dimethylphenyl, 3,5-difluorophenyl,
2-methoxyphenyl, 3-methoxyphenyl, 4-methoxyphenyl,
2,4-difluorophenyl, 4-pyridinyl, 3-pyridinyl, 3-ethoxycarbonyl-phenyl
3-methoxycarbonyl-phenyl, phenyl, 4-hydroxyphenyl,
3-hydroxyphenyl, 3,4 difluorophenyl, pyrimidin-5-yl,
2-methylpyriminin-5-yl, 6-fluoropyridin-2-yl,
5-fluoropyridin-2-yl, 4-fluoropyridin-2-yl, 2-fluoropyridin-4-yl,
3-fluoropyridin-4-yl.

In one embodiment of the invention preferred groups R⁴ are hydrogen, methyl and fluoro.

In another embodiment of the invention the invention relates to Stereoisomers of general formula la 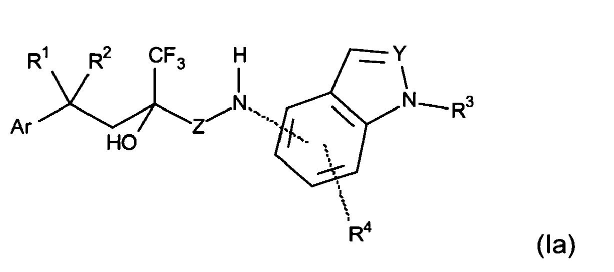 in which
- Z: is selected from the group consisting of CH₂ and CO
- Y: is selected from the group consisting of CH and N
- Ar: is selected from the group consisting of a phenyl, a pyridinyl or a pyrimidinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁵, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁵ means hydrogen or C₁-C₄-alkyl
- R¹, R²: are independently selected from the group consisting of a hydrogen atom or a (C₁-C₄)-alkyl group,
or
- R¹, R²: together form a C₃-C₆ cycloalkyl-ring
- R³: is selected from the group consisting of a phenyl, a pyrimidinyl and a pyridinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁶ means hydrogen or C₁-C₄-alkyl
- R⁴: is selected from the group consisting of halogen, cyano, nitro, (C₁-C₅)-halo-alkyl, (C₁-C₅)alkoxy, (C₁-C₅)halo-alkoxy and COOR⁶, in which R⁶ has the above identified meaning.

Preferred groups R⁴ in the embodiment according to formula la are F, Cl or a CF₃- group.

Preferred groups Ar in the embodiment according to formula la is the group consisting of
2,3-dihydro-1-benzofuran-7-yl, 5-fluoro-2-methoxy-phenyl,
5-fluoro-2-hydroxy-phenyl, 4-Chloro-2-methoxyphenyl, 4-Fluoro-2-methoxyphenyl,
5-Chloro-2-methoxyphenyl, 4-Chloro-2-hydroxyphenyl,
4-Fluoro-2-hydroxyphenyl, 5-Chloro-2-hydroxyphenyl,
2,3-Dihydrobenzofuran-4-yl, 2,3-Dihydrobenzofuran-5-yl,
2,3-Dihydro-5-fluorobenzofuran-7-yl,
2,3-Dihydro-5-chlorobenzofuran-7-yl,
Benzo[1,3]dioxol-4-yl, 4-Fluoro-Benzo[1,3]dioxol-4-yl,
5-Fluoro-Benzo[1,3]dioxol-4-yl, 4-Chloro-Benzo[1,3]dioxol-4-yl,
5-Chloro-Benzo[1,3]dioxol-4-yl, 2,4-Difluorophenyl, 2,5-Difluorophenyl
2-Chloro-5-fluorophenyl, 5-Chloro-2-fluorophenyl.

Preferred groups R³ in the embodiment according to formula la is the group consisting of:
4-fluorophenyl, 3-fluorophenyl, 2-fluorophenyl,
4-chlorophenyl, 3-chlorophenyl, 2-chlorophenyl,
6-fluoropyridin-3-yl, 5-fluoropyridin-3-yl, 4-fluoropyridin-3-yl,
6-chloropyridin-3-yl, 5-chloropyridin-3-yl, 4-chloropyridin-3-yl,
2-fluoropyridin-4-yl, 3-fluoropyridin-4-yl, 2-chloropyridin-4-yl,
3-chloropyridin-4-yl, 4-methylphenyl, 3,5-dimethylphenyl,
3,5-difluorophenyl, 2-methoxyphenyl, 3-methoxyphenyl,
4-methoxyphenyl, 2,4-difluorophenyl, 4-pyridinyl, 3-pyridinyl,
3-ethoxycarbonyl-phenyl, 3-methoxycarbonyl-phenyl,
phenyl, 4-hydroxyphenyl, 3-hydroxyphenyl,
3,4-difluorophenyl, pyrimidin-5-yl, 2-methylpyriminin-5-yl
6-fluoropyridin-2-yl, 5-fluoropyridin-2-yl, 4-fluoropyridin-2-yl
2-fluoropyridin-4-yl, 3-fluoropyridin-4-yl.

In addition, the invention relates to the use of the compounds of general formula I or la for the production of pharmaceutical agents as well as their use for the production of pharmaceutical agents for treating inflammatory diseases.

Due to the presence of asymmetry centers the compounds of general formula I or Ia according to the invention may have several stereoisomers. Subjects of this invention are all possible enantiomers or diastereomers, both as racemates and in enantiomer-pure form.

The compounds according to the invention can also be present in the form of salts with physiologically compatible anions, for example in the form of hydrochlorides, sulfates, nitrates, phosphates, pivalates, maleates, fumarates, tartrates, benzoates, mesylates, citrates or succinates.

The compounds can be produced by the various processes that are generally described in WO98/54159, WO00/32584, WO02/10143 and WO05/003098). The expert in the field does know from these sources together with the information provided below and his general knowledge how to synthesize the compounds according to the present invention.
A) An aminoindazole or indole of general formula (II), in which Ar, B, R¹ and R² have the meanings that are indicated for formula (I), is converted with an arylboronic acid of the general formula (III), in which under R³ has the meaning that is indicated for formula (I), under catalysis of copper or palladium (P. Lam et al. Syn. Lett. 2000; 5, 674 - 76; H. Sano et al, Biorg. Med. Chem. 2005, 13, 3079-91) to compounds of the general formula (I) according to the invention. 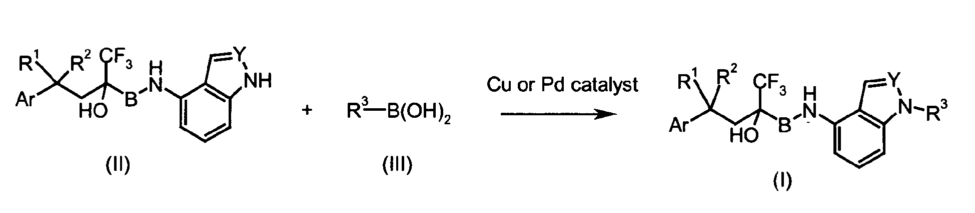
B) Title compound (I) can also be synthesized with by reductive amination of an aldehyde of general formula (IV), in which Ar, R¹ and R² have the meanings that are indicated for formula (I), and an amino-1-arylindazole or indole of general formula (V), in which Y and R³ are defined as described above, according to the methods that are known to one skilled in the art, whereby, e.g., sodium cyanoborohydride, sodium triacetoxy borohydride, sodium borohydride or hydrogen is considered as a reducing agent under palladium catalysis and e.g.acetic acid or titanium tetra alcoholates are considered as acidic catalysts for the imine formation. 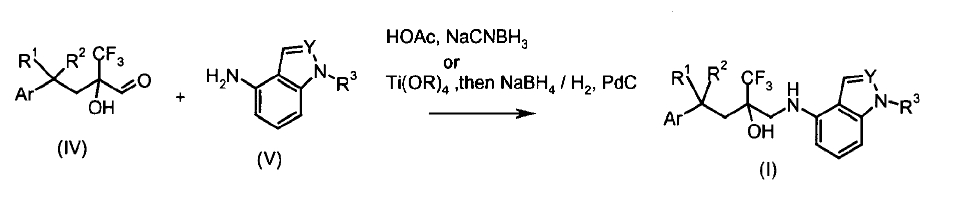
C) An epoxide of general formula (VI), in which Ar, R¹ and R² have the above-indicated meaning, can be opened by an amino-1-arylindazole or indole of general formula (V), in which Y and R³ are defined as described above, according to the methods that are known to one skilled in the art, whereby, e.g., DABCO or boron trifluorid are present during the reaction (WO 2005/003098), to give the amine (Ia). 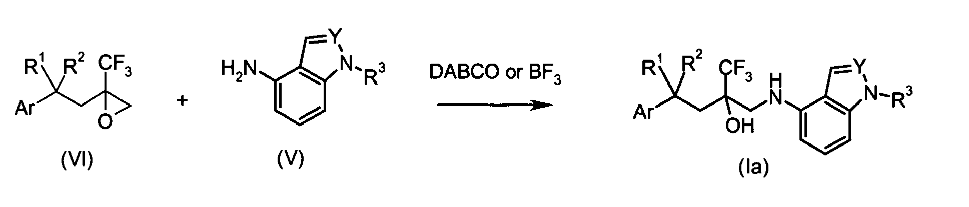
D) Another method consists in reacting compound (VII) (WO 2005/003098), in which Ar, B, R¹ and R² have the above-indicated meaning, under base catalysis, e.g., in the presence of tertiary amine bases or alkali carbonates or alkali hydroxides, or under transition metal catalysis, e.g., palladium catalysis (J. P. Wolfe, S. Wagaw, J.-F. Marcoux, S. L. Buchwald Acc. Chem. Res. 1998, 31, 805; J. F. Hartwig Acc. Chem. Res. 1998, 31, 852), with a halogenated arylindazole or arylindole of general formula (VIII), into title compound (I). 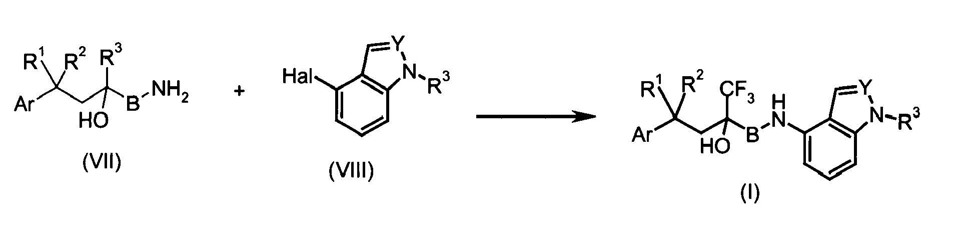
E) A hydroxy acid of general formula (IX), in which Ar, R¹ and R² have the meanings that are indicated for formula (I), is converted with an amino-1-arylindazole or indole of general formula (V), in which Y and R³ are defined as described above, in the way that is known to one skilled in the art. For example, amide (Ib) is obtained with use of dehydrating coupling reagents, as they are known from peptide chemistry, e.g., dicyclohexylcarbodiimide or 1-(3-dimethylaminopropyl)-3-ethylcarbodiimides, or by upstream conversion of the acid into an acid chloride, e.g., with thionyl chloride or POCl₃ and subsequent reaction with the amino-1-arylindazole or indole.

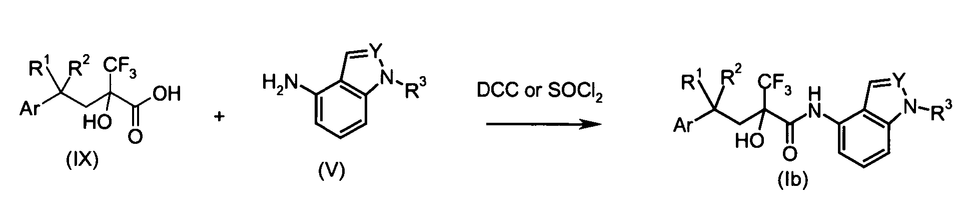

If the compounds according to the invention are present as racemic mixtures, they can be separated into pure, optically active forms according to the methods of racemate separation that are familiar to one skilled in the art. For example, the racemic mixtures can be separated by chromatography on an even optically active carrier material (CHIRALPAK AD^{®}) into the pure isomers. It is also possible to esterify the free hydroxy group in a racemic compound of general formula I with an optically active acid and to separate the diastereoisomeric esters that are obtained by fractionated crystallization or by chromatography, and to saponify the separated esters in each case to the optically pure isomers. As an optically active acid, for example, mandelic acid, camphorsulfonic acid or tartaric acid can be used.

The binding of the substances to the glucocorticoid receptor (GR) and other steroid hormone receptors (mineral corticoid receptor (MR), progesterone receptor (PR) and androgen receptor (AR)) is examined with the aid of recombinantly produced receptors. Cytosol preparations of Sf9 cells, which had been infected with recombinant baculoviruses, which code for the GR, are used for the binding studies. In comparison to reference substance [³H]-dexamethasone, the substances show a high to very high affinity to GR. IC₅₀(GR) = 11,5 nM was thus measured for the compound from Example A16.

As an essential, molecular mechanism for the anti-inflammatory action of glucocorticoids, the GR-mediated inhibition of the transcription of cytokines, adhesion molecules, enzymes and other pro-inflammatory factors is considered. This inhibition is produced by an interaction of the GR with other transcription factors, e.g., AP-1 and NF-kappa-B (for a survey, see Cato, A. C. B., and Wade, E., BioEssays 18, 371-378, 1996).

The compounds of general formula I or Ia according to the invention inhibit the secretion of cytokine IL-8 into the human monocyte cell line THP-1 that is triggered by lipopolysaccharide (LPS). The concentration of the cytokines was determined in the supernatant by means of commercially available ELISA kits. The compound from Example A16 showed an inhibition IC₅₀(IL8) = 0,3 nmol (94% eff. relative to dexamethasone).

The anti-inflammatory action of the compounds of general formula I or Ia was tested in the animal experiment by tests in the croton oil-induced inflammation in rats and mice (J. Exp. Med. (1995), 182, 99-108). To this end, croton oil in ethanolic solution was applied topically to the animals' ears. The test substances were also applied topically or systemically at the same time or two hours before the croton oil. After 16-24 hours, the ear weight was measured as a yardstick for inflammatory edema, the peroxidase activity as a yardstick for the invasions of granulocytes, and the elastase activity as a yardstick for the invasion of neutrophilic granulocytes. In this test, the compounds of general formula I or Ia inhibit the three above-mentioned inflammation parameters both after topical administration and after systemic administration.

One of the most frequent undesirable actions of a glucocorticoid therapy is the so-called "steroid diabetes" [cf., Hatz, H. J., Glucocorticoide: Immunologische Grundlagen, Pharmakologie und Therapierichtlinien [Glucocorticoids: Immunological Bases, Pharmacology and Therapy Guidelines], Wissenschaftliche Verlagsgesellschaft mbH, Stuttgart, 1998]. The reason for this is the stimulation of gluconeogenesis in the liver by induction of the enzymes responsible in this respect and by free amino acids, which are produced from the degradation of proteins (catabolic action of glucocorticoids). A key enzyme of the catabolic metabolism in the liver is tyrosinamino transferase (TAT). The activity of this enzyme can be determined from liver homogenates by photometry and represents a good measurement of the undesirable metabolic actions of glucocorticoids. To measure the TAT induction, the animals are sacrificed 8 hours after the test substances are administered, the livers are removed, and the TAT activity is measured in the homogenate. In this test, at doses in which they have an anti-inflammatory action, the compounds of general formula I induce little or no tyrosinamino transferase.

Because of their anti-inflammatory and, in addition, anti-allergic, immunosuppressive and antiproliferative action, the compounds of general formula I or la according to the invention can be used as medications for treatment or prophylaxis of the following pathologic conditions in mammals and humans: In this case, the term "DISEASE" stands for the following indications:
(i) Lung diseases that are accompanied by inflammatory, allergic and/or proliferative processes:
   - Chronic, obstructive lung diseases of any origin, primarily bronchial asthma
   - Bronchitis of different origins
   - All forms of restrictive lung diseases, primarily allergic alveolitis,
   - All forms of pulmonary edema, primarily toxic pulmonary edema
   - Sarcoidoses and granulomatoses, especially Boeck's disease
(ii) Rheumatic diseases/autoimmune diseases/joint diseases that are accompanied by inflammatory, allergic and/or proliferative processes:
   - All forms of rheumatic diseases, especially rheumatoid arthritis, acute rheumatic fever, polymyalgia rheumatica
   - Reactive arthritis
   - Inflammatory soft-tissue diseases of other origins
   - Arthritic symptoms in the case of degenerative joint diseases (arthroses)
   - Traumatic arthritides
   - Collagenoses of any origin, e.g., systemic lupus erythematodes, sclerodermia, polymyositis, dermatomyositis, Sjögren's syndrome, Still's syndrome, Felty's syndrome
(iii) Allergies that are accompanied by inflammatory and/or proliferative processes:
   - All forms of allergic reactions, e.g., Quincke's edema, hay fever, insect bites, allergic reactions to pharmaceutical agents, blood derivatives, contrast media, etc., anaphylactic shock, urticaria, contact dermatitis
(iv) Vascular inflammations (vasculitides)
   - Panarteritis nodosa, temporal arteritis, erythema nodosum
(v) Dermatological diseases that are accompanied by inflammatory, allergic and/or proliferative processes:
   - Atopic dermatitis (primarily in children)
   - Psoriasis
   - Pityriasis rubra pilaris
   - Erythematous diseases, triggered by different noxae, e.g., radiation, chemicals, burns, etc.
   - Bullous dermatoses
   - Diseases of the lichenoid group,
   - Pruritis (e.g., of allergic origin)
   - Seborrheal eczema
   - Rosacea
   - Pemphigus vulgaris
   - Erythema exudativum multiforme
   - Balanitis
   - Vulvitis
   - Hair loss such as alopecia areata
   - Cutaneous T-cell lymphoma
(vi) Kidney diseases that are accompanied by inflammatory, allergic and/or proliferative processes:
   - Nephrotic syndrome
   - All nephritides
(vii) Liver diseases that are accompanied by inflammatory, allergic and/or proliferative processes:
   - Acute liver cell decomposition
   - Acute hepatitis of different origins, e.g., viral, toxic, pharmaceutical agent-induced
   - Chronic aggressive hepatitis and/or chronic intermittent hepatitis
(viii) Gastrointestinal diseases that are accompanied by inflammatory, allergic and/or proliferative processes:
   - Regional enteritis (Crohn's disease)
   - Colitis ulcerosa
   - Gastritis
   - Reflux esophagitis
   - Ulcerative colitis of other origins, e.g., native sprue
(ix) Proctologic diseases that are accompanied by inflammatory, allergic and/or proliferative processes:
   - Anal eczema
   - Fissures
   - Hemorrhoids
   - Idiopathic proctitis
(x) Eye diseases that are accompanied by inflammatory, allergic and/or proliferative processes:
   - Allergic keratitis, uveitis, iritis
   - Conjunctivitis
   - Blepharitis
   - Optic neuritis
   - Chorioiditis
   - Sympathetic ophthalmia
(xi) Diseases of the ear-nose-throat area that are accompanied by inflammatory, allergic and/or proliferative processes:
   - Allergic rhinitis, hay fever
   - Otitis externa, e.g., caused by contact dermatitis, infection, etc.
   - Otitis media
(xii) Neurological diseases that are accompanied by inflammatory, allergic and/or proliferative processes:
   - Cerebral edema, primarily tumor-induced cerebral edema
   - Multiple sclerosis
   - Acute encephalomyelitis
   - Meningitis
   - Various forms of convulsions, e.g., infantile nodding spasms
(xiii) Blood diseases that are accompanied by inflammatory, allergic and/or proliferative processes:
   - Acquired hemolytic anemia
   - Idiopathic thrombocytopenia
(xiv) Tumor diseases that are accompanied by inflammatory, allergic and/or proliferative processes:
   - Acute lymphatic leukemia
   - Malignant lymphoma
   - Lymphogranulomatoses
   - Lymphosarcoma
   - Extensive metastases, mainly in breast, bronchial and prostate cancers
(xv) Endocrine diseases that are accompanied by inflammatory, allergic and/or proliferative processes:
   - Endocrine orbitopathy
   - Thyreotoxic crisis
   - De Quervain's thyroiditis
   - Hashimoto's thyroiditis
   - Basedow's disease
(xvi) Organ and tissue transplants, graft-versus-host disease
(xvii) Severe shock conditions, e.g., anaphylactic shock, systemic inflammatory response syndrome (SIRS)
(xviii) Substitution therapy in:
   - Innate primary suprarenal insufficiency, e.g., congenital adrenogenital syndrome
   - Acquired primary suprarenal insufficiency, e.g., Addison's disease, autoimmune adrenalitis, meta-infective tumors, metastases, etc.
   - Innate secondary suprarenal insufficiency, e.g., congenital hypopituitarism
   - Acquired secondary suprarenal insufficiency, e.g., meta-infective tumors, etc.
(xix) Vomiting that is accompanied by inflammatory, allergic and/or proliferative processes:
   - e.g., in combination with a 5-HT3 antagonist in cytostatic-agent-induced vomiting
(xx) Pains of inflammatory origins, e.g., lumbago.

Moreover, the compounds of general formula I according to the invention can be used for treatment and prophylaxis of additional pathologic conditions that are not mentioned above, for which synthetic glucocorticoids are now used (see in this respect Hatz, H. J., Glucocorticoide: Immunologische Grundlagen, Pharmakologie und Therapierichtlinien, Wissenschaftliche Verlagsgesellschaft mbH, Stuttgart, 1998).

All previously mentioned indications (i) to (xx) are described in more detail in Hatz, H. J., Glucocorticoide: Immunologische Grundlagen, Pharmakologie und Therapierichtlinien, Wissenschaftliche Verlagsgesellschaft mbH, Stuttgart, 1998.

For the therapeutic actions in the above-mentioned pathologic conditions, the suitable dose varies and depends on, for example, the active strength of the compound of general formula I, the host, the type of administration, and the type and severity of the conditions that are to be treated, as well as the use as a prophylactic agent or therapeutic agent.

In addition, the invention provides:
(i) The use of one of the compounds of formula I or la according to the invention or mixture thereof for the production of a medication for treating a DISEASE;
(ii) A process for treating a DISEASE, said process comprises an administration of an amount of the compound according to the invention, in which the amount suppresses the disease and in which the amount of compound is given to a patient who requires such a medication;
(iii) A pharmaceutical composition for treating a DISEASE, said treatment comprises one of the compounds according to the invention or mixture thereof and at least one pharmaceutical adjuvant and/or vehicle.

In general, satisfactory results can be expected in animals when the daily doses comprise a range of 1 µg to 100,000 µg of the compound according to the invention per kg of body weight. In the case of larger mammals, for example the human, a recommended daily dose lies in the range of 1 µg to 100,000 µg per kg of body weight. Preferred is a dose of 10 to 30,000 µg per kg of body weight, and more preferred is a dose of 10 to 10,000 µg per kg of body weight. For example, this dose is suitably administered several times daily. For treating acute shock (e.g., anaphylactic shock), individual doses can be given that are significantly above the above-mentioned doses.

The formulation of the pharmaceutical preparations based on the new compounds is carried out in a way that is known in the art by the active ingredient being processed with the vehicles that are commonly used in galenicals, fillers, substances that influence decomposition, binding agents, moisturizers, lubricants, absorbents, diluents, flavoring correctives, coloring agents, etc., and converted into the desired form of administration. In this case, reference is made to Remington's Pharmaceutical Science, 15th Edition, Mack Publishing Company, East Pennsylvania (1980).

For oral administration, especially tablets, coated tablets, capsules, pills, powders, granulates, lozenges, suspensions, emulsions or solutions are suitable.

For parenteral administration, injection and infusion preparations are possible.

For intra-articular injection, correspondingly prepared crystal suspensions can be used.

For intramuscular injection, aqueous and oily injection solutions or suspensions and corresponding depot preparations can be used.

For rectal administration, the new compounds can be used in the form of suppositories, capsules, solutions (e.g., in the form of enemas) and ointments both for systemic and for local treatment.

For pulmonary administration of the new compounds, the latter can be used in the form of aerosols and inhalants.

For local application to eyes, outer ear channels, middle ears, nasal cavities, and paranasal sinuses, the new compounds can be used as drops, ointments and tinctures in corresponding pharmaceutical preparations.

For topical application, formulations in gels, ointments, fatty ointments, creams, pastes, powders, milk and tinctures are possible. The dosage of the compounds of general formula I or la should be 0.01 %-20% in these preparations to achieve a sufficient pharmacological action.

The invention also comprises the compounds of general formula I or la according to the invention as therapeutic active ingredients.

In addition, the compounds of general formula I or la according to the invention are part of the invention as therapeutic active ingredients together with pharmaceutically compatible and acceptable adjuvants and vehicles.

The invention also comprises a pharmaceutical composition that contains one of the pharmaceutically active compounds according to the invention or mixtures thereof or a pharmaceutically compatible salt thereof and a pharmaceutically compatible salt or pharmaceutically compatible adjuvants and vehicles.

The compounds according to the invention can optionally also be formulated and/or administered in combination with other active ingredients.

The invention therefore also relates to combination therapies or combined compositions, in which a compound of general formula (I) or (Ia) or a pharmaceutically acceptable salt thereof, or a pharmaceutical composition that contains a compound of general formula (I) or (Ia) or a pharmaceutically acceptable salt thereof, is administered either simultaneously (optionally in the same composition) or in succession together with one or more pharmaceutical agents for treating one of the above-mentioned pathologic conditions. For example, for treatment of rheumatoid arthritis, osteoarthritis, COPD (chronic obstructive lung disease), asthma or allergic rhinitis, a compound of general formula (I) of this invention can be combined with one or more pharmaceutical agents for treating such a condition. When such a combination is administered by inhalation, the pharmaceutical agent that is to be combined can be selected from the following list:
- A PDE4 inhibitor including an inhibitor of the PDE4D isoform,
- A selective β.sub2.adrenoceptor agonist, such as, for example, metaproterenol, isoproterenol, isoprenaline, albuterol, salbutamol, formoterol, salmeterol, terbutaline, orcipresnaline, bitolterol mesylate, pirbuterol or indacaterol;
- A muscarine receptor antagonist (for example, an M1, M2 or M3 antagonist, such as, for example, a more selective M3 antagonist), such as, for example, ipratropium bromide, tiotropium bromide, oxitropium bromide, pirenzepine or telenzepine;
- A modulator of the chemokine receptor function (such as, for example, a CCR1 receptor antagonist); or
- An inhibitor of the p38 kinase function.

For another subject of this invention, such a combination with a compound of general formula (I) or (Ia) or a pharmaceutically acceptable salt thereof is used for treatment of COPD, asthma or allergic rhinitis and can be administered by inhalation or orally in combination with xanthine (such as, for example, aminophylline or thyeophylline), which also can be administered by inhalation or orally.

### Experimental Part:

The various aspects of the invention described in this application are illustrated by the following examples which are not meant to limit the invention in any way.

### Example A1

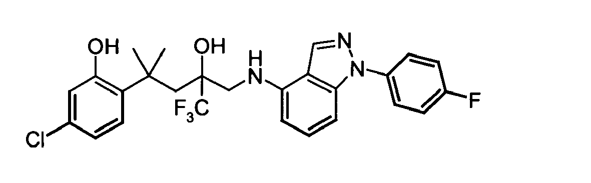

### 4-(4-Chloro-2-methoxyphenyl)-4-methyl-1-{[1-(4-fluorophenyl)1H-indazol-4-yl]amino}-2-(trifluoromethyl)-pentan-2-ol

30 mg (0,07 mmol) 4-(4-chloro-2-methoxyphenyl)-4-methyl-1-[(1H-indazol-4-yl)amino]-2-(trifluoromethyl)-pentan-2-ol (WO 2005/003098), 29,4 mg (0,21 mmol) 4-fluorophenyl boronic acid and 13 mg (0,064 mmol) copper acetate are stirred in 0,02 ml pyridine and 2 ml dichloromethane for 16 hours. The reaction mixture is treated with 3 g of silica gel and remaining solvent is removed in a vacuum. After chromatography on silica gel with hexane-ethyl acetate (50%), 6 mg of the desired product is obtained. ¹H-NMR (CDCl₃); δ = 1.45 (s, 3H), 1.59 (s, 3H), 2.24 (d, 1 H), 2.84 (d, 1 H), 3.25 (d, 1 H), 3.41 (d, 1 H), 5.73 (d, 1 H), 6.64 (d, 1 H), 6.87 (dd, 1 H), 6.94 (d, 1 H), 7.15 (d, 1 H), 7.16 (dd, 2H), 7.27 (d, 1 H), 7.58 (dd, 2H), 7.99 (s, 1H).

### Example A10

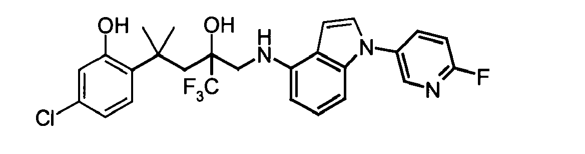

### 4-(4-Chloro-2-hydroxyphenyl)-4-methyl-1-{[1-(6-fluoropyridin-3-yl)1H-indol-4-yl]amino}-2-(trifluoromethyl)pentan-2-ol

Analogously to Example A1, 4-(4-Chloro-2-hydroxyoxyphenyl)-1-(1 H-indol-4-ylamino)-4-methyl-2-(trifluoromethyl)pentan-2-ol (WO 2005/003098) can be reacted with (6-fluoropyridin-3-yl)boronic acid under copper acetate catalysis.

### Example A11

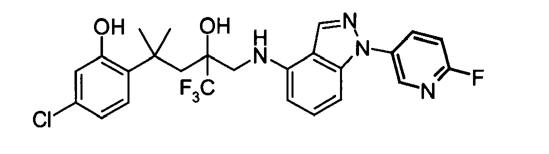

### 4-(4-Chloro-2-hydroxyphenyl)-4-methyl-1-{[1-(6-fluoropyridin-3-yl)indazol-4-yl]amino}-2-(trifluoromethyl)pentan-2-ol

Analogously to Example A1, 4-(4-Chloro-2-hydroxyoxyphenyl)-1-(1H-indazol-4-ylamino)-4-methyl-2-(trifluoromethyl)pentan-2-ol (WO 2005/003098) can be reacted with (6-fluoropyridin-3-yl)boronic acid under copper acetate catalysis.

### Example A12

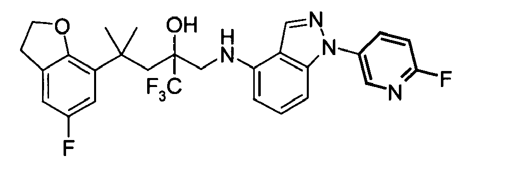

### 4(-2,3-Dihydro-5-fluorobenzofuran-7-yl)-4-methyl-1-{[1-(6-fluoropyridin-3-yl)indazol-4-yl]amino}-2-(trifluoromethyl)pentan-2-ol

### 4-Amino-1-(6-fluoropyrimidin-3-yl)indazole

0,5 g (3,06 mmol) of 4-nitroindazole *(*Chem. Ber. 37,1904, 2583) 860 mg (6,13 mmol) of (6-fluoropyrimidin-3-yl)boronic acid and 557 mg (2,79 mmol) copper acetate are stirred in 0,5 ml pyridine and 20 ml dichloromethane for 16 hours. The reaction mixture is treated with 5 g of silica gel and remaining solvent is removed in a vacuum. After chromatography on silica gel with hexane-ethyl acetate (50-100%), 280 mg of 4-nitro-1-(6-fluoropyridin-3-yl)indazole is obtained. 280 mg (1,08 mmol) of 4-nitro-1-(6-fluoropyridin-3-yl)indazole is dissolved in 8 ml methanol, 4 ml 2-propanole and 8 ml THF. 50 mg of Raney nickel is added, and the mixture is shaken for 3 hours under a hydrogen atmosphere at normal pressure. The catalyst is removed from the solution by means of filtration on Celite, whereby washing is continued with methanol and ethyl acetate, and it is concentrated by evaporation. After chromatography on silica gel with hexan - ethyl acetate (50%), 180 mg of the desired 5-Amino-1-(6-fluoropyridin-3-yl)indazole is obtained. ¹H-NMR (CDCl₃); δ = 6.47 (d, 1 H), 7.05 (d, 1 H), 7.12 (dd, 1 H), 7.27 (dd, 1 H), 8.16 (ddd, 1 H), 8.18 (s, 1 H), 8.62 (m, 1 H).

0.1 ml (0,32 mmol) of titanium tetra *t*-butylate and 0,1 ml of acetic acid are added to 50 mg (0,16 mmol)of 4-(2,3-Dihydro-5-fluorobenzofuran-7-yl)-2-hydroxy-4-methyl-2-(trifluoromethyl)pentanal and 36 mg (0,16 mmol) of 4-amino-1-(6-fluoropyridin-3-yl)indazol in 4 ml of toluene, and the mixture is heated over 2.5 hours to 100°C. After cooling, it is poured into water, and vigorous stirring is continued. The suspension is filtered through Celite, and is thoroughly rewashed with ethyl acetate. The phases of the filtrate are separated, and it is extracted again with ethyl acetate. It is dried on sodium sulfate, and the solvent is removed in a vacuum to yield 83 mg crude 4-(2,3-dihydro-5-fluorobenzofuran-7-yl)-4-methyl-1-{[(4-fluorophenyl)indazole-4-yl]imino}-4-methyl-2-(trifluoromethyl)pentan-2-ol. The crude Imine is dissolved in 4 ml of methanol and cooled to 5°C. 12 mg (10 mmol) sodium borohydrate are added and the mixture is stirred 2,5 hours at room temperature. The reaction mixture is carefully mixed with saturated NH₄Cl while being cooled in an ice bath, and diluted with water and ethyl acetate. Phases are separated and the aqueous phase is two times extracted with ethyl acetate. The combined organics are washed with brine, it is dried on sodium sulfate, and the solvent is removed in a vacuum. The crude product are purified by thin layer chromatography on silica gel with hexane-ethyl acetate (20%) to yield 62 mg not reduced imine and 13 mg of the desired product. ¹H-NMR (CDCl₃); δ = 1.44 (s, 3H), 1.54 (s, 3H), 2.32 (d, 1H), 2.62 (d, 1H), 3.10 (dd, 2H), 3.18 (dd, 1 H), 3.27 (d, 1H), 3.42 (d, 1H), 4.54 (dd, 1 H), 4.61 (dd, 1 H), 5.89 (d, 1H), 6.86 (dd, 1 H), 6.90 (dd, 1H), 7.01 (d, 1 H), 7.11 (dd, 1 H), 7.22 (t, 1 H), 8.02 (s,1 H), 8.13 (ddd, 1 H), 8.59 (m, 1 H).

### Example A13

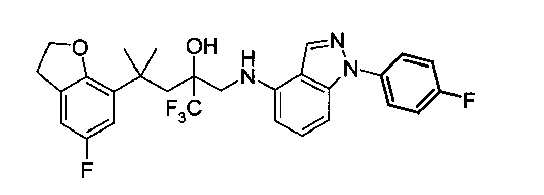

### 4-(2,3-Dihydro-5-fluorobenzofuran-7-yl)-4-methyl-1-{[1-(4-fluorophenyl)1H-indazol-4-yl]amino}-2-(trifluoromethyl)pentan-2-ol

### 4-Amino- 1-(4-fluorophenyl)indazole

0,5 g (3,06 mmol) of 4-nitroindazole (*Chem. Ber. 37*,**1904**, 2583) 860 mg (6,13 mmol) of 4-fluorophenyl boronic acid and 558 mg (2,79 mmol) copper acetate are stirred in 0,5 ml pyridine and 20 ml dichloromethane for 16 hours. The reaction mixture is treated with 5 g of silica gel and remaining solvent is removed in a vacuum. After chromatography on silica gel with hexane - ethyl acetate (50-100%), 600 mg of 4-nitro-1-(4-fluorophenyl)indazole is obtained. 300 mg (1,17 mmol) of 4-nitro-1-(4-fluorophenyl)indazole is dissolved in 10 ml methanol, 5 ml 2-propanole and 10 ml THF. 100 mg of Raney nickel is added, and the mixture is shaken for 3 hours under a hydrogen atmosphere at normal pressure. Catalyst is removed from the solution by means of filtration on Celite, whereby washing is continued with methanol and ethyl acetate, and it is concentrated by evaporation. After chromatography on silica gel with ethyl acetate-ethanol (0-10%), 240 mg of the desired 4-Amino-1-(4-fluorophenyl)indazole is obtained. ¹H-NMR (CDCl₃); δ = 4.22 (br, 2H), 6.43 (d, 1H), 7.05 (d, 1 H), 7.21 (dd, 2H), 7.22 (dd, 1 H), 7.68 (dd, 2H), 8.13 (s, 1 H).

50 mg (0,16 mmol)of 4-(2,3-Dihydro-5-fluorobenzofuran-7-yl)-2-hydroxy-4-methyl-2-(trifluoromethyl)pentanal and 36 mg (0,16 mmol) of 4-amino-1-(4-fluorophenyl)indazole are reacted, as described in Example A12, to form 4-(2,3-Dihydro-5-fluorobenzofuran-7-yl)-4-methyl-1-{(4-fluorophenyl)1H-indazole-4-yl]imino}-4-methyl-2-(trifluoromethyl)pentan-2-ol in the presence of titanium tetra *t-*butylate and acetic acid. Further reduction with sodium borohydride in methanol yields 15 mg of the desired product after chromatography on silica gel. ¹H-NMR (CDCl₃); δ = 1.44 (s, 3H), 1.55 (s, 3H), 2.29 (d, 1 H), 2.64 (d, 1 H), 3.09 (dd, 1 H), 3.19 (dd, 1 H), 3.26 (d, 1 H), 3.42 (d, 1 H), 4.55 (dd, 1 H), 4.60 (dd, 1 H), 5.83 (d, 1 H), 6.85 (dd, 1 H), 6.91 (dd, 1 H), 7.01 (d, 1 H), 7.19 (m, 3H), 7.63 (dd, 2H), 7.98 (s, 1H).

### Example A16

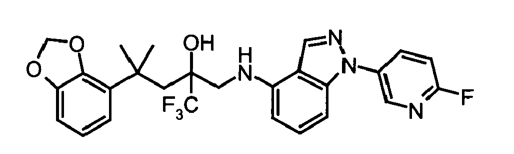

### 4-(Benzo[1,3]dioxol-4-yl)-4-methyl-1-[1-(6-fluoropyridin-3-yl)indazol-4-ylaminol-2-(trifluoromethyl)pentan-2-ol

Analogously to Example A1, 20 mg (0,047 mmol) of 4-(benzo[1,3]dioxol-4-yl)-4-methyl-1-[(1H-indazol-4-yl)amino]-2-(trifluoromethyl)pentan-2-ol (WO 2005/003098) are reacted with 20 mg (0,14 mmol) of (6-fluoropyridin-3-yl)boronic acid under copper acetate catalysis. Preparative thin layer chromatography on silica gel plates with hexane ethyl acetate (33%) yields 10 mg of the desired product. ¹H-NMR (CDCl₃); δ = 1.48 (s, 3H), 1.59 (s, 3H), 2.32 (d, 1 H), 2.53 (d, 1 H), 3.0 (s, 1 H), 3.27 (d, 1 H), 3.45 (d, 1 H), 5.90 (d, 1 H), 5.93 (s, 1 H), 5.94 (s, 1 H), 6.83 (dd, 1 H), 6.92 (m, 2H), 7.00 (d, 1 H), 7.10 (dd, 1 H), 7.20 (t, 1 H), 8.02 (s,1H), 8.12 (ddd, 1 H), 8.59 (m, 1H).

### Example A17

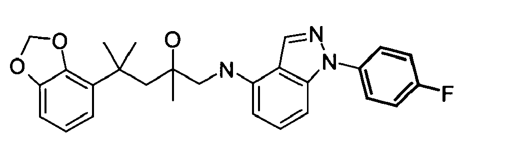

### 4-(Benzo[1,3]dioxol-4-yl)-4-methyl-1-[1-(4-fluorophenyl)indazol-4-ylamino]-2-(trifluoromethyl)pentan-2-ol

Analogously to Example A1, 19,8 mg (0,047 mmol) of 4-(benzo[1,3]dioxol-4-yl)-4-methyl-1-(indazol-4-ylamino)-2-(trifluoromethyl)pentan-2-ol (WO 2005/003098) are reacted with 19,7 mg (0,14 mmol) of 4-fluorophenyl boronic acid under copper acetate catalysis. Preparative thin layer chromatography on silica gel plates with hexane ethyl acetate (33%) yields 21 mg of the desired product. ¹H-NMR (CDCl₃); δ = 1.48 (s, 3H), 1.59 (s, 3H), 2.31 (d, 1 H), 2.54 (d, 1 H), 3.0 (s, 1 H), 3.27 (d, 1 H), 3.45 (d, 1 H), 5.89 (d, 1 H), 5.93 (s, 1 H), 5.94 (s, 1 H), 6.83 (dd, 1 H), 6.92 (m, 2H), 7.02 (d, 1 H), 7.16 (dd, 2H), 7.21 (t, 1 H), 7.64 (dd, 1 H), 7.99 (s,1 H).

### Example A18

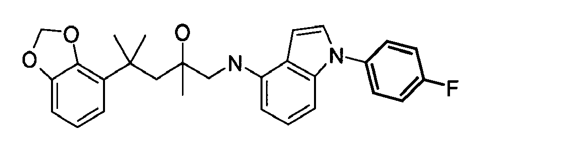

### 4-(Benzo[1,3]dioxol-4-yl)-4-methyl-1-[1-(4-fluorophenyl)indol-4-ylamino]-2-(trifluoromethyl)pentan-2-ol

Analogously to Example A1, 4-(benzo[1,3]dioxol-4-yl)-1-(1 H-indol-4-ylamino)-4-methyl-2-(trifluoromethyl)pentan-2-ol can be reacted with 4-fluorophenyl boronic acid under copper acetate catalysis.

### Example A19

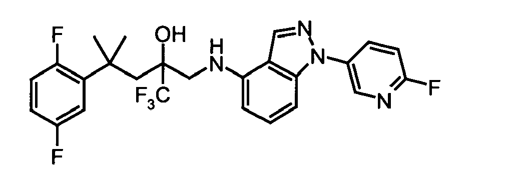

### 4-(2,5-Difluorophenyl)-4-methyl-1-{[(6-fluoropyridin-3-yl)indazol-4-ylamino}-2-(trifluoromethyl)pentan-2-ol

Analogously to Example A1, 4-(2,5-difluorophenyl)-1-[(1 H-indazol-4-yl)amino]-4-methyl-2-(trifluoromethyl)pentan-2-ol can be reacted with (6-fluoropyridin-3-yl)boronic acid under copper acetate catalysis.

## Claims

1. Stereoisomers of general formula I 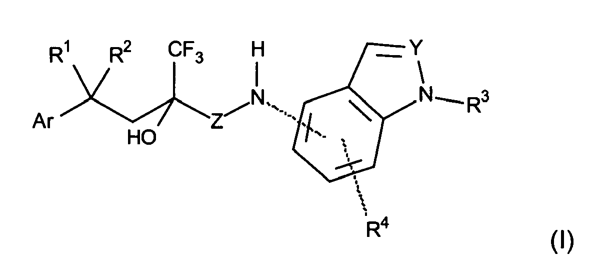 in which
Z is selected from the group consisting of CH₂ and CO
Y is selected from the group consisting of CH and N
Ar is selected from the group consisting of a phenyl, a pyridinyl or a pyrimidinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁵, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁵ means hydrogen or C₁-C₄-alkyl
provided that if B is CH₂ and Y is N and R¹, R² are both methyl then Ar is not 2,3-dihydro-1-benzofuran-7-yl, 5-fluoro-2-methoxy-phenyl or 5-fluoro-2-hydroxy-phenyl
R¹, R² are independently selected from the group consisting of a hydrogen atom or a (C₁-C₄)-alkyl group,
or
R¹, R² together form a C₃-C₆ cycloalkyl-ring
R³ is selected from the group consisting of a phenyl, a pyrimidinyl and a pyridinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁶ means hydrogen or C₁-C₄-alkyl
R⁴ is selected from the group consisting of hydrogen, halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶,
in which R⁶ has the above identified meaning.

2. Stereoisomers according to claim 1, in which Ar is selected from the group consisting of 4-Chloro-2-methoxyphenyl, 4-Fluoro-2-methoxyphenyl, 5-Chloro-2-methoxyphenyl, 4-Chloro-2-hydroxyphenyl,
4-Fluoro-2-hydroxyphenyl, 5-Chloro-2-hydroxyphenyl,
2,3-Dihydrobenzofuran-4-yl, 2,3-Dihydrobenzofuran-5-yl,
2,3-Dihydro-5-fluorobenzofuran-7-yl,
2,3-Dihydro-5-chlorobenzofuran-7-yl,
Benzo[1,3]dioxol-4-yl, 4-Fluoro-Benzo[1,3]dioxol-4-yl,
5-Fluoro-Benzo[1,3]dioxol-4-yl, 4-Chloro-Benzo[1,3]dioxol-4-yl,
5-Chloro-Benzo[1,3]dioxol-4-yl, 2,4-Difluorophenyl, 2,5-Difluorophenyl
2-Chloro-5-fluorophenyl, 5-Chloro-2-fluorophenyl.

3. Stereoisomers according to claim 1, in which at least one of the groups R¹ and R² is selected from the group consisting of hydrogen, methyl, ethyl or in which R¹ and R² together are selected from the group cyclopropyl, cyclobutyl, cyclopentyl or cyclohexyl.

4. Stereoisomers according to claim 1, in which R¹ and R² are both methyl.

5. Stereoisomers according to claim 1, in which Z is a CH₂ group.

6. Stereoisomers according to claim 1, in which Y is a N atom.

7. Stereoisomers according to claim 6, in which the link between the amine or amide and the indazole is formed via the the 4- and the 5-position of he indazole. Most preferred is the 4-position.Y is a N atom.

8. Stereoisomers according to claim 7, in which the link between the amine or amide and the indazole is formed via the the 4-position of he indazole.

9. Stereoisomers according to claim 1, in which R³ is selected from the group comprising:
4-fluorophenyl, 3-fluorophenyl, 2-fluorophenyl,
4-chlorophenyl, 3-chlorophenyl, 2-chlorophenyl,
6-fluoropyridin-3-yl, 5-fluoropyridin-3-yl, 4-fluoropyridin-3-yl,
6-chloropyridin-3-yl, 5-chloropyridin-3-yl, 4-chloropyridin-3-yl,
2-fluoropyridin-4-yl, 3-fluoropyridin-4-yl, 2-chloropyridin-4-yl,
3-chloropyridin-4-yl, 4-methylphenyl, 3,5-dimethylphenyl,
3,5-difluorophenyl, 2-methoxyphenyl, 3-methoxyphenyl,
4-methoxyphenyl, 2,4-difluorophenyl, 4-pyridinyl, 3-pyridinyl,
3-ethoxycarbonyl-phenyl, 3-methoxycarbonyl-phenyl,
phenyl, 4-hydroxyphenyl, 3-hydroxyphenyl,
3,4-difluorophenyl, pyrimidin-5-yl, 2-methylpyriminin-5-yl
6-fluoropyridin-2-yl, 5-fluoropyridin-2-yl, 4-fluoropyridin-2-yl
2-fluoropyridin-4-yl, 3-fluoropyridin-4-yl.

10. Stereoisomers according to claim 1, in which R⁴ is selected from the group consisting of hydrogen, methyl and fluoro.

11. Use of the stereoisomers according to at least one of claims 1-5 for the manufacture of pharmaceutical agents.

12. Use of the stereoisomers according to at least one of claims 1-5 for the manufacture of pharmaceutical agents for treating inflammatory diseases.

13. Stereoisomers of general formula la 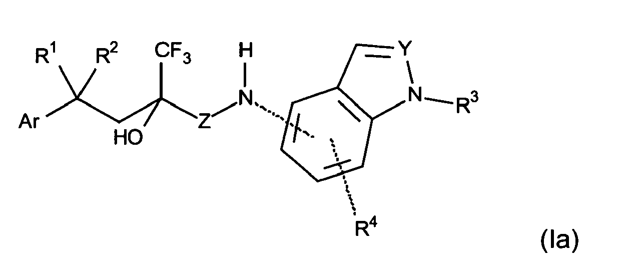 in which
Z is selected from the group consisting of CH₂ and CO
Y is selected from the group consisting of CH and N
Ar is selected from the group consisting of a phenyl, a pyridinyl or a pyrimidinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁵, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁵ means hydrogen or C₁-C₄-alkyl
R¹, R² are independently selected from the group consisting of a hydrogen atom or a (C₁-C₄)-alkyl group,
or
R¹, R² together form a C₃-C₆ cycloalkyl-ring
R³ is selected from the group consisting of a phenyl, a pyrimidinyl and a pyridinyl rest
which may have 1-4 substituents independently selected from the group consisting of halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁶, and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-, -N(C₁-C₃-alkyl)-(CH₂)ₚ₊₁-, and -NH-N=CH-,
in which p = 1 or 2, and in which R⁶ means hydrogen or C₁-C₄-alkyl
R⁴ is selected from the group consisting of halogen, cyano, nitro, (C₁-C₅)-halo-alkyl, (C₁-C₅)alkoxy, (C₁-C₅)halo-alkoxy and COOR⁶, in which R⁶ has the above identified meaning.

14. Stereoisomers acording to claim 13 in which R⁴ is F, Cl or a CF₃- group.

15. Stereoisomers acording to claim 13 or 14 in which Ar is selected from the group consisting of
2,3-dihydro-1-benzofuran-7-yl, 5-fluoro-2-methoxy-phenyl,
5-fluoro-2-hydroxy-phenyl, 4-Chloro-2-methoxyphenyl,
4-Fluoro-2-methoxyphenyl,
5-Chloro-2-methoxyphenyl, 4-Chloro-2-hydroxyphenyl,
4-Fluoro-2-hydroxyphenyl, 5-Chloro-2-hydroxyphenyl,
2,3-Dihydrobenzofuran-4-yl, 2,3-Dihydrobenzofuran-5-yl,
2,3-Dihydro-5-fluorobenzofuran-7-yl,
2,3-Dihydro-5-chlorobenzofuran-7-yl,
Benzo[1,3]dioxol-4-yl, 4-Fluoro-Benzo[1,3]dioxol-4-yl,
5-Fluoro-Benzo[1,3]dioxol-4-yl, 4-Chloro-Benzo[1,3]dioxol-4-yl,
5-Chloro-Benzo[1,3]dioxol-4-yl, 2,4-Difluorophenyl, 2,5-Difluorophenyl
2-Chloro-5-fluorophenyl, 5-Chloro-2-fluorophenyl.

16. Stereoisomers acording to at least one of claims 13 to 15 in which R³ is selected from the group consisting of:
4-fluorophenyl, 3-fluorophenyl, 2-fluorophenyl,
4-chlorophenyl, 3-chlorophenyl, 2-chlorophenyl,
6-fluoropyridin-3-yl, 5-fluoropyridin-3-yl, 4-fluoropyridin-3-yl,
6-chloropyridin-3-yl, 5-chloropyridin-3-yl, 4-chloropyridin-3-yl,
2-fluoropyridin-4-yl, 3-fluoropyridin-4-yl, 2-chloropyridin-4-yl,
3-chloropyridin-4-yl, 4-methylphenyl, 3,5-dimethylphenyl,
3,5-difluorophenyl, 2-methoxyphenyl, 3-methoxyphenyl,
4-methoxyphenyl, 2,4-difluorophenyl, 4-pyridinyl, 3-pyridinyl,
3-ethoxycarbonyl-phenyl, 3-methoxycarbonyl-phenyl,
phenyl, 4-hydroxyphenyl, 3-hydroxyphenyl,
3,4-difluorophenyl, pyrimidin-5-yl, 2-methylpyriminin-5-yl
6-fluoropyridin-2-yl, 5-fluoropyridin-2-yl, 4-fluoropyridin-2-yl
2-fluoropyridin-4-yl, 3-fluoropyridin-4-yl.

17. Use of the stereoisomers according to at least one of claims 13-16 for the manufacture of pharmaceutical agents.

18. Use of the stereoisomers according to at least one of claims 13-16 for the manufacture of pharmaceutical agents for treating inflammatory diseases.
